# EUROPÄISCHE PATENTSCHRIFT

(11) **EP 3 178 743 B1**
(45) Veröffentlichungstag und Bekanntmachung des Hinweises auf die Patenterteilung: **07.04.2021**
(21) Anmeldenummer: 16197448.0
(22) Anmeldetag: 07.11.2016
(51) Int. Cl.: B65B 39/00, A61M 39/00, F16L 11/04, A61M 25/00, A61M 5/00, A61J 1/20, F16L 11/10, B65B 3/04, A61M 39/08, A61M 39/12, B65B 39/02, B65B 3/00

(54) **SCHLAUCHSET UND ABFÜLLNADEL ZUR VERWENDUNG IN EINEM SCHLAUCHSET ZUM ABFÜLLEN EINES FLIESSFÄHIGEN MEDIUMS, INSBESONDERE EINES PHARMAZEUTIKUMS**
HOSE SET AND FILLING NEEDLE FOR USE IN A HOSE SET FOR FILLING A FLOWABLE MEDIUM, IN PARTICULAR A PHARMACEUTICAL MEDIUM
TUYAU EN KIT ET AIGUILLE D'ASPIRATION DESTINÉE À ÊTRE UTILISÉE DANS UN TUYAU EN KIT POUR SOUTIRER UN MILIEU COULANT, EN PARTICULIER UN PRODUIT PHARMACEUTIQUE

(30) Priorität: 13.11.2015 DE 102015222397
(43) Veröffentlichungstag der Anmeldung: 14.06.2017
(73) Patentinhaber: RAUMEDIC AG, 95213 Münchberg (DE)
(72) Erfinder: JORDAN, Philipp, 95447 Bayreuth (DE); HAGER, Steffen, 95233 Helmbrechts (DE); ERHARD, Dominik, 95326 Kulmbach (DE); ADELUNG, Rainer, 24147 Kiel (DE); MEß, Kristin, 24119 Kronshagen (DE); PAULOWICZ, Ingo, 24114 Kiel (DE); SCHÜTT, Fabian, 24118 Kiel (DE)
(74) Vertreter: Rau, Schneck & Hübner Patentanwälte Rechtsanwälte PartGmbB

(56) Entgegenhaltungen:
- EP-A1- 1 450 092
- WO-A2-2008/103484
- WO-A2-2015/010687
- DE-A1-102006 001 178
- DE-A1-102011 076 938
- DE-A1-102013 010 509
- DE-A1-102013 104 195
- DE-U1-202010 004 551
- US-A- 4 347 874
- US-A1- 2009 227 954
- US-A1- 2011 270 019
- US-A1- 2012 192 987
- US-A1- 2013 056 130
- US-A1- 2013 085 413
- US-A1- 2014 283 940
- XIN JIN ET AL: "Joining the Un-Joinable: Adhesion Between Low Surface Energy Polymers Using Tetrapodal ZnO Linkers", ADVANCED MATERIALS, Bd. 24, Nr. 42, 8. November 2012 (2012-11-08), Seiten 5676-5680, XP055324022, DE ISSN: 0935-9648, DOI: 10.1002/adma.201201780

## Beschreibung

Die vorliegende Patentanmeldung nimmt die Priorität der deutschen Patentanmeldung DE 10 2015 222 397.3 in Anspruch, deren Inhalt durch Bezugnahme hierin aufgenommen wird.

Die Erfindung betrifft eine Abfüllnadel zur Verwendung in einer Schlauchschicht zum Abfüllen eines fließfähigen Mediums, insbesondere eines Pharmazeutikums.

Eine derartige Abfüllnadel ist bekannt aus der WO 2008/103 484 A2.

Aus der US 9,192,754 B2 ist ein Silikonschlauch mit geringer Permeabilität bekannt. Die US 2006/0025753 A1 beschreibt einen Blasen-Katheter. Die US 2009/0053084 A1 beschreibt ein Silikon-Schlauchsegment für eine peristaltische Pumpe. Die US 4,347,874 beschreibt eine Abfüll-Baugruppe mit einem peristaltischen Pumpschlauch und einer Abfüllnadel. Die DE 10 2013 010509 A1 beschreibt ein Schlauchsystem zum Anschluss an eine Abfüllanlage zur Befüllung von Behältnissen mit pharmazeutischen Flüssigkeiten. Die EP 1 450 092 A1 beschreibt einen mehrlagigen Schlauch zur Fluidförderung. Die US 2012/0192987 A1 beschreibt einen Silikonschlauch, beschichtet mit einem als Gasbarriere wirkenden Material. Die DE 10 2006 001178 A1 beschreibt ein Verfahren zum Abfüllen von Flüssigkeiten und eine Vorrichtung zur Durchführung des Verfahrens. In dem Fachartikel "Joining the Un-Joinable: Adhesion Between Low Surface Energy Polymers Using Tatrapodal ZnO Linkers" von Jin et al., Adv. Mater. 2012, 24, 5676 bis 5680 ist Haftverbund aus PTFE und PDNS beschrieben. Die WO 2015/010687 A2 beschreibt ein Polymerlaminat und ein Verfahren zu seiner Herstellung. Die DE 10 2013 104195 A1 beschreibt ein optoelektronisches Bauelement und ein Verfahren zu seiner Herstellung. Die US 2013/0056130 A1 beschreibt ein System und ein Verfahren zur Erfassung einer Verbindung zwischen zwei Komponenten. Die US 2009/0227954 A1 beschreibt eine aus mehreren Abschnitten aufgebaute Verbindungseinrichtung für elastische Schläuche. Die US 2014/0283940 A1 beschreibt einen durch Extrusion hergestellten Mehrlagenschlauch. Die DE 10 2011 077938 A1 beschreibt einen Konnektor zur fluiddichten Verbindung mindestens zweier fluidführender Komponenten sowie eine Fluidtransfer-Baugruppe mit mindestens einem derartigen Konnektor. Die US 2011/0270019 A1 beschreibt eine implantierbare Einrichtung zum Schutz eines Schlauches.

Es ist eine Aufgabe der vorliegenden Erfindung, eine Abfüllnadel zu schaffen, die kostengünstig herstellbar ist.

Diese Aufgabe ist erfindungsgemäß gelöst durch eine Abfüllnadel mit den im Anspruch 1 angegebenen Merkmalen.

Erfindungsgemäß wurde erkannt, dass es möglich ist, eine Abfüllnadel mit einer ein inneres Schlauchlumen vorgebenden inneren Schlauchschicht aus Silikon auszuführen. Eine solche Abfüllnadel kann als Einwegprodukt genutzt werden, so dass eine aufwendige Reinigung entfällt. Die Abfüllnadel kann so ausgeführt sein, dass das fließfähige Medium, insbesondere ein Pharmazeutikum, ausschließlich in Kontakt mit der inneren Silikon-Schlauchschicht kommt. Eine unerwünschte Kontamination des Pharmazeutikums ist somit verhindert. Die Abfüllnadel besteht insgesamt ausschließlich aus Kunststoffmaterial.

Die äußere Versteifungs-Schlauchschicht führt zu einer formstabilen Abfüllnadel. Eine aufwendige sonstige Armierung kann entfallen. Die gesamte Abfüllnadel kann aus Kunststoff gefertigt sein. Die Versteifungs-Schlauchschicht kann aus einem Polyolefin gefertigt sein. Die Versteifungs-Schlauchschicht kann aus einem Thermoplasten gefertigt sein. Die Versteifungs-Schlauchschicht ist aus Polypropylen (PP), Polyethylen (PE), insbesondere aus Low Density Polyethylen (LDPE), Polycarbonat (PC) oder Polysulfon (PSU) gefertigt.

Ein Haftvermittler nach Anspruch 2 verhindert eine unerwünschte Delamination der Abfüllnadel bzw. eine unerwünschte axiale Relativverlagerung der inneren Schlauchschicht zur äußeren Versteifungs-Schlauchschicht.

Mechanisch die beiden Schlauchschichten verbindenden Haftvermittlungskörper nach Anspruch 3 haben sich zur Erzeugung einer sicheren Haftung zwischen den beiden Schlauchschichten als besonders geeignet herausgestellt. Bei den Haftvermittlungskörpern kann es sich um Tetrapoden handeln. Die Haftvermittlungskörper können aus Zinkoxid (ZnO) gefertigt sein. Derartige Haftvermittlungskörper sind grundsätzlich bekannt aus dem Fachartikel von Jin et al., Adv. Mater. 2012, 24, 5676 bis 5680. Die Haftvermittlungskörper können eine typische Abmessung im Bereich zwischen 10 µm und 100 µm, insbesondere im Bereich zwischen 10 µm und 40 µm aufweisen. Eine Verbindungswirkung dieser mechanisch verbindenden Haftvermittlungskörper beruht nicht im Wesentlichen auf Adhäsion, sondern aufgrund von Formschlussbeiträgen und beruht insbesondere auf einer mechanischen Verankerung bzw. Verkrallung. Hierdurch ist eine sichere Verbindung zwischen den Schlauchschichten möglich, ohne dass sekundäre Bindungkräfte Dipol-Dipol-Kräfte oder Wasserstoffbrücken zum Einsatz kommen müssen. Die Haftvermittlungskörper können direkt mit mit der mindestens einen Silikon-Komponente einerseits und mit der mindestens einen weiteren Polymer-Komponente andererseits mechanisch in Kontakt kommen. Alternativ ist es möglich, dass zumindest einige oder alle Haftvermittlungskörper von einer der Komponenten des Mehrkomponenten-Kunststoffkörpers vollständig umschlossen werden, wobei sich eine mechanische Verbindung der beiden Komponenten über einen Formschlussbeitrag zwischen dem von der einen Komponente umhüllten Haftvermittlungskörper und der anderen Komponente im Grenzbereich zwischen den beiden Komponenten aufgrund der Form des Haftvermittlungskörpers ergibt. Eine mechanische Verbindung im Sinne der Anmeldung liegt insbesondere dann vor, wenn ein Formschlussbeitrag, der für einen Zusammenhalt zwischen den Komponenten des Mehrkomponenten-Kunststoffkörpers sorgt, aufgrund von Hinterschnitten der Haftvermittlungskörper resultiert.

Am Nadelende der Abfüllnadel kann die innere Schlauchschicht über eine äußere Versteifungs-Schlauchschicht überstehen, wobei die äußere Versteifungs-Schlauchschicht aus einem Kunststoff-Versteifungsmaterial ist und das innere Schlauchlumen umgibt. Ein derartiger Überstand der inneren Schlauchschicht am Nadelende stellt sicher, dass am Nadelende nur die innere Schlauchschicht mit dem fließfähigen Pharmazeutikum in Kontakt kommt. Dieser Überstand kann im Bereich zwischen 1 mm und 5 mm liegen. Ein solcher Überstand kann durch nachträgliches Konfektionieren der Abfüllnadel erzeugt werden. Ein solcher Überstand kann auch ein vorteilhaftes Abtropfen des Pharmazeutikums vom Nadelende begünstigen.

Am Konnektorende der Abfüllnadel kann die innere Schlauchschicht über die äußere Versteifungs-Schlauchschicht überstehen. Ein derartiger Überstand der inneren Schlauchschicht am Konnektorende vereinfacht eine Konnektierung der Abfüllnadel so, dass das Pharmazeutikum am Konnektorende ebenfalls ausschließlich nur mit der inneren Silikon-Schlauchschicht der Abfüllnadel in Kontakt kommt.

Die Vorteile eines Schlauchsets nach Anspruch 4 entsprechen denen, die vorstehend unter Bezugnahme auf die erfindungsgemäße Abfüllnadel bereits erläutert wurden.

Ein Aufstülpen der inneren Schlauchschicht nach Anspruch 5 oder ein Einschieben der inneren Schlauchschicht nach Anspruch 6 sind Varianten der Verbindung der inneren Schlauchschicht mit der Konnektor-Komponente, die sicherstellen können, dass das fließfähige Pharmazeutikum am Konnektorende ausschließlich mit der inneren Silikon-Schlauchschicht in Kontakt kommt.

Ausführungsbeispiele der Erfindung werden nachfolgend anhand der Zeichnung näher erläutert. In dieser zeigen:
- Fig. 1: schematisch einen Mehrkomponenten-Kunststoffkörper mit einer Silikonschicht und einer Polymerschicht aus einem weiteren unpolaren Polymer und einem Haftvermittler zwischen diesen beiden Schichten;
- Fig. 2: eine Ausschnittsvergrößerung II aus Fig. 1, die Details des die beiden Schichten mechanisch verbindenden Haftvermittlers zeigt;
- Fig. 3 bis 6: schematische Querschnitte eines Schichtaufbaus verschiedener Ausführungen von Mehrkomponenten-Kunststoffkörpern, die jeweils als Mehrschichtschläuche ausgeführt sind;
- Fig. 7: eine Seitenansicht einer Abfüllnadel zur Verwendung in ei-nem Schlauchset zum Abfüllen eines Mediums, insbesondere eines fließfähigen Pharmazeutikums;
- Fig. 8: einen Querschnitt gemäß Linie VIII-VIII in Fig. 7; und
- Fig. 9 bis 11: Ausführungen der Abfüllnadel nach Fig. 7 mit verschiedenen Varianten einer fluiddichten Verbindung einer Konnektorseite der Abfüllnadel mit verschiedenen Ausführungen einer Konnektorkomponente zu einer Fülleinrichtung des Schlauchsets.

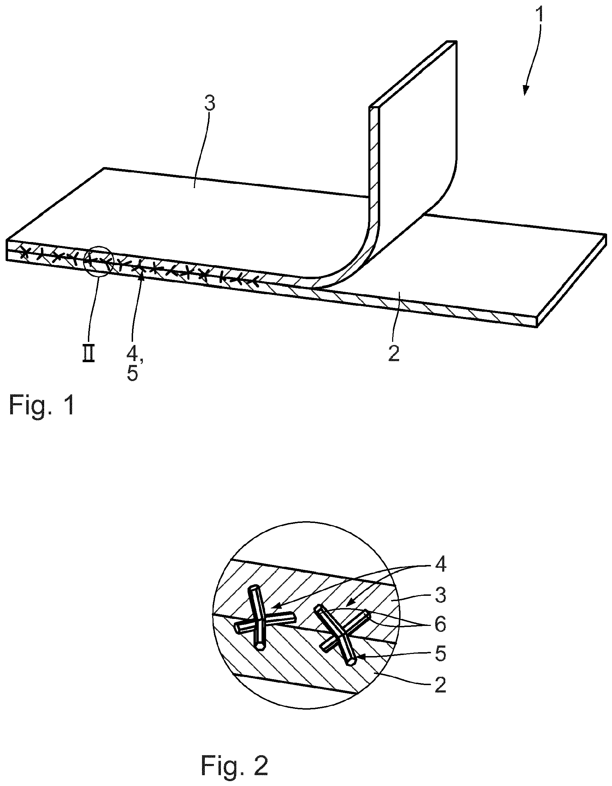

Fig. 1 zeigt eine Ausführung eines Mehrkomponenten-Kunststoffkörpers 1 am Beispiel eines Zweischicht-Kunststoffkörpers. Der Kunststoffkörper 1 hat eine Silikon-Komponente in Form einer Silikonschicht 2 und eine weitere Polymer-Komponente in Form einer Polymerschicht 3. Diese weitere Polymerschicht 3 ist aus einem unpolaren Polymer. Die weitere Polymerschicht 3 ist nicht aus Silikon. Bei dem weiteren Polymer der Polymerschicht 3 kann es sich um Polyethylen oder Polypropylen, insbesondere aus Low Density Polyethylen (LDPE) handeln. Die weitere Polymer-Komponente 3 kann aus einem Thermoplasten, insbesondere aus einem thermoplastischen Elastomer sein. Bei dem weiteren Polymer kann es sich um ein unpolares Polyolefin handeln.

Beispiele für unpolare Polymere sind Polyethylen (PE), Polypropylen (PP), Polystyrol (PS) oder Polytetrafluorethylen (PTFE). Beispiele für wenig polare Polymere, die im Sinne dieser Anmeldung ebenfalls noch als unpolare Polymer zu verstehen sind, sind Copolymerisate aus Ethylen und ungesättigten Estern (z. B. EVAC) oder Polyphenylenether (PPE).

Der Mehrkomponenten-Kunststoffkörper 1 kann in der Medizin oder in der Pharmazie zur Anwendung kommen.

Zwischen der Silikon-Komponente 2 und der weiteren Polymer-Komponente 3 ist ein Haftvermittler 4 angeordnet. Der Haftvermittler 4 weist die beiden Komponenten 2, 3, also die beiden Schichten, mechanisch miteinander verbindende Haftvermittlungskörper 5 auf. Die Haftvermittlungskörper 5 sind aus Zinkoxid (ZnO). Die Haftvermittlungskörper 5 haben die Form von Tetrapoden. Die ZnO-Tetrapoden haben eine typische Abmessung im Bereich zwischen 1 µm und 100 µm, insbesondere im Bereich zwischen 10 µm und 40 µm. Freie Enden 6 der Haftvermittlungskörper 5 verankern bzw. verkrallen sich einerseits in bzw. mit der Silikonschicht 2 und andererseits in bzw. mit der weiteren Polymerschicht 3. Auf diese Weise ergibt sich eine mechanische Verbindung zwischen den beiden Schichten 2, 3, also zwischen der Silikonschicht 2 und der weiteren Polymerschicht 3. Diese mechanische Verbindung hat einen Formschlussbeitrag, beruht also nicht im Wesentlichen auf Adhäsion. Ein Adhäsionsbeitrag dieser Verbindung ist nicht ausgeschlossen.

Bei der in der Ausschnittvergrößerung der Fig. 2 gezeigten Anordnung stehen die Haftvermittlungskörper 5 direkt in Kontakt einerseits mit der Silikonschicht 2 und andererseits mit der weiteren Polymerschicht 3. Alternativ ist es möglich, dass jedenfalls einige oder alle der Haftvermittlungskörper 5 von einer der beiden Komponenten, also entweder der Silikonschicht 2 oder der weiteren Polymerschicht 3, vollständig umhüllt werden, wobei eine mechanische Verbindung zwischen den beiden Schichten 2, 3 dann immer noch aufgrund eines Formschlussbeitrages, erzeugt über die Form der Haftvermittlungskörper, insbesondere über mindestens einen Hinterschnitt resultiert. Beispielsweise können die Haftvermittlungskörper 5 im Bereich eines Endes 6 oder mehrerer Enden 6, die in die Polymerschicht 3 hineinragen, mit dem Silikonmaterial der Silikonschicht 2 überzogen sein.

Zur Herstellung des Mehrkomponenten-Kunststoffkörpers 1 wird zunächst eine Basis-Komponente hergestellt, bei der es sich entweder um die Silikon-Komponente 2 oder um die weitere Polymer-Komponente 3 handelt. Anschließend wird der Haftvermittler 4 auf diese Basis-Komponente 2 oder 3 aufgebracht. Schließlich wird eine Deck-Komponente, bei der es sich dann um die weitere Polymer-Komponente oder um die Silikon-Komponente handeln kann, auf die Basis-Komponente aufgebracht, so dass der Haftvermittler 4 zwischen der Silikon-Komponente 2 und der weiteren Polymer-Komponente 3 zu liegen kommt.

Vor dem Aufbringen des Haftvermittlers 4 auf die Basis-Komponente können die Haftvermittlungskörper 5 des Haftvermittlers 4 in einem Fluid, insbesondere in einem Silikonfluid, dispergiert werden. Anschließend werden die dispergierten Haftvermittlungskörpers 5 auf die Basis-Komponente aufgebracht, bevor wiederum die Deck-Komponente aufgebracht wird.

Vor dem Aufbringen des Haftvermittlers 4 kann ein Silikatisieren einer dem Haftvermittler 4 zugewandten Oberfläche der Basis-Komponente, also beispielsweise der Silikon-Komponente 2, stattfinden.

Nach dem Aufbringen der Deck-Komponente kann ein Heizen eines so erzeugten Roh-Mehrkomponenten-Kunststoffkörpers erfolgen, wodurch insbesondere die Haftvermittlungskörper 5 besser in den Schichten 2, 3 verankert werden. Nach dem Heizen dieses Roh-Mehrkomponenten-Kunststoffkörpers und dem nachfolgenden Abkühlen entsteht dann der fertige Mehrkomponenten-Kunststoffkörper 1.

Die Silikon-Komponente 2 bzw. die weitere Polymer-Komponente 3 können durch Spritzguss hergestellt werden.

Sofern es sich bei dem Mehrkomponenten-Kunststoffkörper um einen Mehrschichtschlauch handelt, was nachfolgend noch erläutert wird, können die Silikon- bzw. Polymer-Komponenten auch durch Extrusion hergestellt bzw. aufgebracht werden.

Zur Aufbringung einer Haftvermittler-Dispersion kann die jeweils vorgefertigte innere Schlauchschicht durch die Haftvermittler-Dispersion hindurchgeführt werden.

Ein Ummanteln der mit dem Haftvermittler beschichteten inneren Schlauchschicht kann mit Hilfe eines Querspritzkopfes erfolgen.

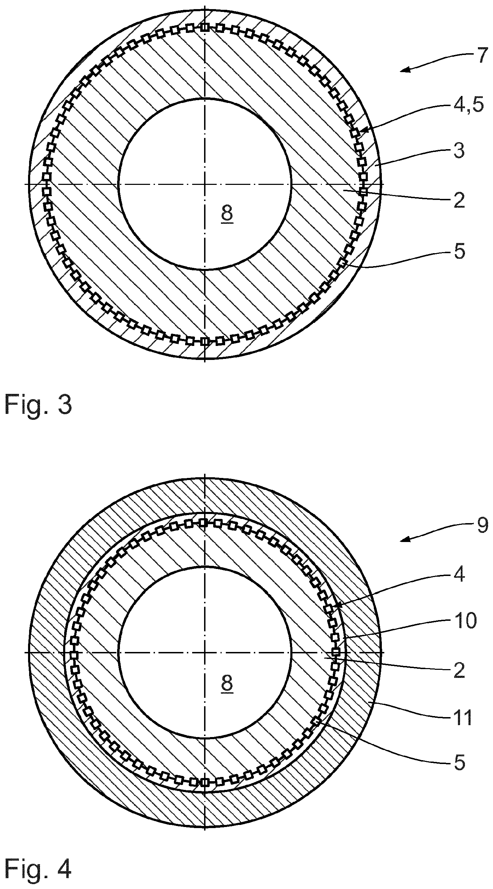

Fig. 3 zeigt eine Ausführung eines Mehrkomponenten-Kunststoffkörpers als Mehrschichtschlauch 7. Die Silikon-Komponente 2 bildet dabei eine Silikon-Schlauchschicht und die weitere Polymer-Komponente 3 eine Polymer-Schlauchschicht. Bei der Ausführung nach Fig. 3 ist die Silikon-Schlauchschicht 2 eine innere Schlauchschicht des Mehrschichtschlauchs 7, die ein Lumen 8 des Mehrschichtschlauchs 7 begrenzt. Die Silikon-Schlauchschicht 3 hat eine Härte von Shore 60 A.

Beim Silikonmaterial der Silikon-Schlauchschicht 3 kann es sich um ein platinvernetztes oder um ein peroxidvernetztes Silikon handeln.

Die Polymer-Schlauchschicht 3 des Mehrschichtschlauchs 7 aus dem weiteren unpolaren Polymer ist eine die Silikon-Schlauchschicht 2 umgebende äußere Schlauchschicht. Zwischen den beiden Schlauchschichten 2, 3 des Mehrschichtschlauchs 7 ist wiederum der Haftvermittler 4 mit den Tetrapoden-Haftvermittlungskörpern 5 angeordnet. Die Schichtstärke der inneren Silikon-Schlauchschicht 2 ist größer als diejenige der äußeren Polymer-Schlauchschicht 3.

Der Mehrschichtschlauch 7 kann als pharmazeutischer Abfüllschlauch zum Einsatz kommen. Die weitere Polymer-Schlauchschicht 3 des Mehrschichtschlauchs 7 kann eingefärbt sein. Die äußere Polymer-Schlauchschicht 3 kann komplett lichtundurchlässig oder kann mit einem im UV-Bereich absorbierenden Farbstoff eingefärbt sein. Der Mehrschichtschlauch 7 kann insgesamt transluzent ausgeführt sein, so dass eine optische Kontrolle des Lumens 8 möglich bleibt.

Die innere Silikon-Schlauchschicht 2 kann aus einem platinvernetzendem Silikonkautschuk gebildet sein. Die innere Silikon-Schlauchschicht 2 kann eine silikatisierte Oberfläche aufweisen.

Anhand der Fig. 4 wird nachfolgend eine weitere Ausführung eines Mehrschichtschlauchs 9 erläutert. Komponenten und Funktionen, die denjenigen entsprechen, die vorstehend unter Bezugnahme auf die Fig. 1 bis 3 bereits erläutert wurden, tragen die gleichen Bezugsziffern und Bezeichnungen und werden nicht nochmals im Einzelnen diskutiert.

Der Mehrschichtschlauch 9 umfasst eine innerste Silikonschicht 2, die von einer Zwischen-Schlauchschicht 10 aus einem weiteren Polymer umgeben ist. Die Zwischen-Schlauchschicht 10 stellt die weitere Polymer-Komponente des Mehrschichtschlauchs 9 dar. Zwischen der Zwischen-Schlauchschicht 10 und der innersten Silikon-Schlauchschicht 2 ist der Haftvermittler 4 mit den Haftvermittlungskörpern 5 angeordnet. Die Zwischen-Schlauchschicht 10 ist umgeben von einer äußeren Schlauchschicht 11, die wiederum als Silikon-Schlauchschicht ausgeführt ist. Bei der äußeren Silikon-Schlauchschicht 11 handelt es sich um eine Schlauchschicht aus UV-vernetzendem Silikon. Ein thermischer Vernetzungsschritt für die äußere Silikon-Schlauchschicht 11 ist dann nicht erforderlich. Vor dem Aufbringen der äußeren Silikon-Schlauchschicht 11 kann die Zwischen-Schlauchschicht 10 bei der Herstellung des Mehrschichtschlauchs 9 zur Verbesserung der Anhaftung der äußeren Silikon-Schlauchschicht 11 funktionalisiert werden. Diese Funktionalisierung der Zwischen-Schlauchschicht 10 kann durch eine Korona- oder durch eine Plasmabehandlung erfolgen. Die Zwischen-Schlauchschicht 10 hat eine Schichtstärke, die kleiner ist als die Schichtstärken der Silikon-Schlauchschichten 2 und 11 des Mehrschichtschlauchs 9.

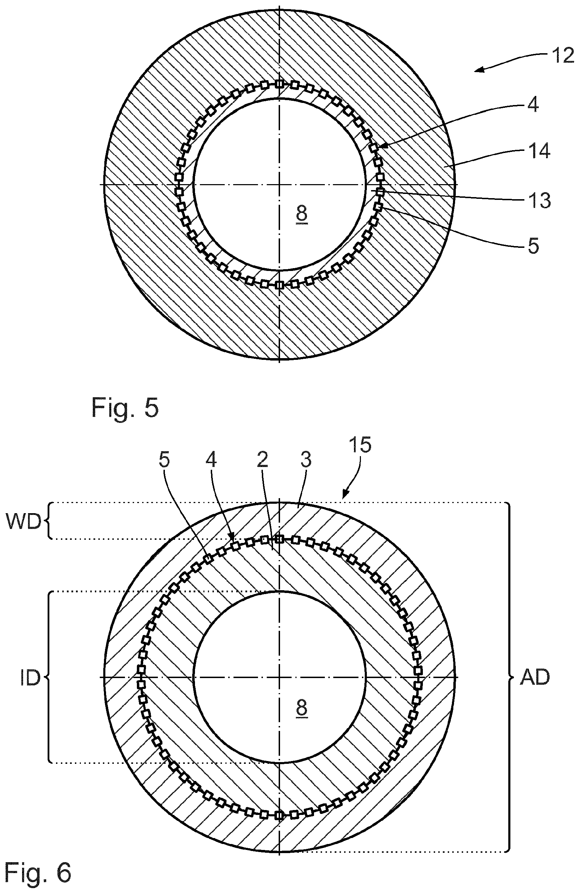

Anhand der Fig. 5 wird nachfolgend eine weitere Ausführung eines Mehrschichtschlauchs 12 erläutert. Komponenten und Funktionen, die denjenigen entsprechen, die vorstehend unter Bezugnahme auf die Fig. 1 bis 4 bereits erläutert wurden, tragen die gleichen Bezugsziffern und Bezeichnungen und werden nicht nochmals im Einzelnen diskutiert.

Beim Mehrschichtschlauch 12 ist eine innerste Schlauchschicht 13 als die weitere Polymer-Komponente ausgeführt. Diese ist umgeben vom Haftvermittler 4 mit den Haftvermittlungskörpern 5 und weiterhin von einer äußeren Schlauchschicht 14, die die Silikon-Komponente des MehrschichtSchlauchs 12 darstellt. Auch bei der äußeren Silikon-Schlauchschicht 14 handelt es sich um eine Schicht aus UV-vernetzendem Silikon. Die Schichtstärke der inneren Polymer-Schlauchschicht 13 ist kleiner als diejenige der äußeren Silikon-Schlauchschicht 14.

Bei der Extrusion der Mehrschichtschläuche 7, 9 und 12 wird zunächst die innerste Schlauchschicht 2, 13 extrudiert und anschließend eine Haftvermittler-Dispersion mit den Haftvermittlungskörpern 5 aufgebracht, woraufhin dann die äußere Schlauchschicht 3, 14 oder die Zwischen-Schlauchschicht 11 per Extrusion aufgebracht wird. Beim Dreischichtschlauch 9 nach Fig. 4 wird im Anschluss hieran die äußere Silikon-Schlauchschicht 11 aufgebracht.

Die Mehrschichtschläuche 9 und 12 können beispielsweise als geruchsdichte Rektalkatheter zum Einsatz kommen.

Aufgrund der Tatsache, dass die Polymer-Schlauchschicht 10 bzw. 13 der Mehrschichtschläuche 9 bzw. 12 weniger stark ist als die beteiligten Silikon-Schlauchschichten 3, 11 bzw. 14 ergibt sich eine gute Flexibilität des jeweiligen Mehrschichtschlauchs 9, 12.

Die Mehrschichtschläuche 9 und 12 sind insgesamt transluzent. Eine optische Kontrolle des Lumens 8 von außen ist möglich.

Die Mehrschichtschläuche 9 und 12 können auch als Pumpschläuche, insbesondere für eine peristaltische Pumpe, zum Einsatz kommen.

Fig. 6 zeigt eine weitere Ausführung eines Mehrschichtschlauchs 15. Komponenten und Funktionen, die denjenigen entsprechen, die vorstehend unter Bezugnahme auf die Fig. 1 bis 5 bereits erläutert wurden, tragen die gleichen Bezugsziffern und Bezeichnungen und werden nicht nochmals im Einzelnen diskutiert.

Der Mehrschichtschlauch 15 entspricht von seinem grundsätzlichen Aufbau dem Mehrschichtschlauch 7 nach Fig. 3. Unterschiede bestehen hauptsächlich bei den Durchmesserverhältnissen. Der Mehrschichtschlauch 15 hat einen Außendurchmesser AD von 5 mm. Ein Innendurchmesser ID beträgt 1,2 mm. Die Wandstärke der äußeren Polymer-Schlauchschicht, WD, beträgt 0,7 mm. Entsprechend beträgt die Wanddicke der inneren Silikon-Schlauchschicht 2 1,2 mm.

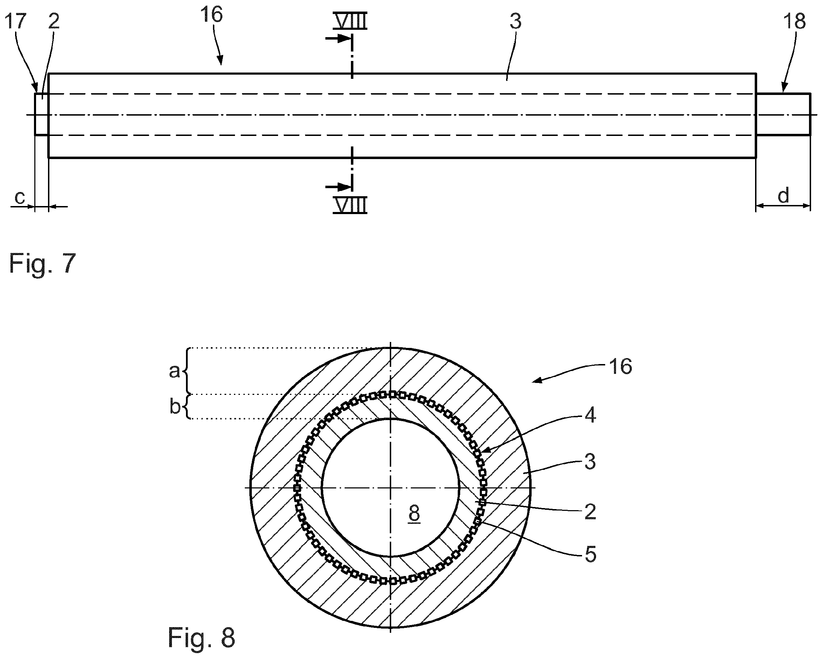

Eine weitere Ausführung eines Mehrkomponenten-Kunststoffkörpers in Form einer Abfüllnadel 16 wird nachfolgend anhand der Fig. 7 bis 11 erläutert. Komponenten und Funktionen, die denjenigen entsprechen, die vorstehend unter Bezugnahme auf die Fig. 1 bis 6 bereits erläutert wurden, tragen die gleichen Bezugsziffern und Bezeichnungen und werden nicht nochmals im Einzelnen diskutiert.

Die Abfüllnadel 16 dient zur Verwendung in einem Schlauchset zum Abfüllen eines fließfähigen Pharmazeutikums. Ein derartiges Schlauchset ist grundsätzlich bekannt aus der WO 2008/103 484 A2.

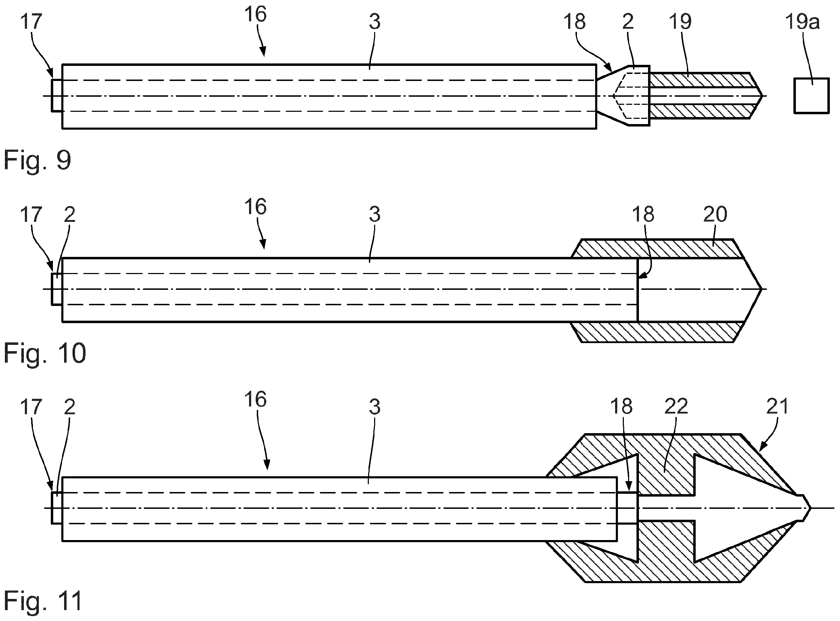

Die Abfüllnadel 16 hat ein in der Fig. 7 linkes Nadelende 17 zum zeitweiligen Einführen in einen Abfüllbehälter. Weiterhin hat die Abfüllnadel 16 ein in der Fig. 7 rechtes, dem Nadelende 17 gegenüberliegendes Konnektorende 18 zum Konnektieren der Abfüllnadel 16 mit einer Fülleinrichtung (vgl. Fig. 9), über die das Pharmazeutikum der Abfüllnadel 16 zuführbar ist. Die Fülleinrichtung ist Teil des Schlauchsets. Weder der Abfüllbehälter noch die Fülleinrichtung sind in der Fig. 7 dargestellt.

Die Abfüllnadel 16 hat eine ein inneres Schlauchlumen 8 vorgebende innere Silikon-Schlauchschicht 2. Vom grundsätzlichen Schichtaufbau her entspricht die Schlauch-Schichtabfolge der Abfüllnadel 16 derjenigen der vorstehend erläuterten Mehrschichtschläuche 7 bzw. 15. Zwischen der inneren Silikon-Schlauchschicht 2 und der äußeren weiteren Polymer-Schlauchschicht 3 liegt wiederum der Haftvermittler 4 mit den Haftvermittlungskörpern 5 vor. Unterschiede zwischen dem Schichtaufbau der Abfüllnadel 16 und denjenigen der Mehrschichtschläuche 7 bzw. 15 ergeben sich wiederum bei den Schichtstärken der beiden Schlauchschichten 2, 3. Eine Schichtstärke a der äußeren, weiteren Polymer-Schlauchschicht 3 ist größer als 0,7 mm. Diese Schichtstärke ist größer oder gleich einer Schichtstärke b der inneren Silikon-Schlauchschicht 2.

Die äußere, weitere Polymer-Schlauchschicht 3 dient als Versteifungs-Schlauchschicht. Die äußere Schlauchschicht 3 kann aus Polypropylen gefertigt sein.

Am Nadelende 17 steht die innere Silikon-Schlauchschicht 2 über die Versteifungs-Schlauchschicht 3 längs eines Überstandes c über. Der Überstand c liegt im Bereich zwischen 1 mm und 5 mm.

Am Konnektorende 18 steht die innere Schlauchschicht 2 über die äußere Versteifungs-Schlauchschicht 3 längs eines Überstandes d über. Der Überstand d kann größer sein als der Überstand c.

Die Fig. 9 bis 11 zeigen verschiedene Ausführungen von Konnektor-Komponenten zum Verbinden der Abfüllnadel 16 mit der Fülleinrichtung des Schlauchsets. Diese Fülleinrichtung ist in der Fig. 9 schematisch bei 19a dargestellt.

Bei der Ausführung nach Fig. 9 ist die Konnektor-Komponente als Füllschlauch 19 ausgebildet, der die Abfüllnadel 16 mit der Fülleinrichtung 19a des Schlauchsets verbindet. Beim Füllschlauch 19 handelt es sich um einen Silikonschlauch. Die innere Silikon-Schlauchschicht 2 der Abfüllnadel 16 ist am Konnektorende 18 auf den Füllschlauch 19 aufgestülpt. Im Bereich des Konnektorendes 18 ist die innere Silikon-Schlauchschicht 2 der Abfüllnadel 16 also aufgeweitet und über einen Außenumfang des Füllschlauchs 19 gelegt, so dass der Füllschlauch 19 am Konnektorende 18 abschnittsweise in die Silikon-Schlauchschicht 2 der Abfüllnadel 16 eingeschoben ist.

Bei der Ausführung nach Fig. 10 ist die Konnektor-Komponente zwischen der Abfüllnadel 16 und der Fülleinrichtung des Schlauchsets ebenfalls als Füllschlauch 20 ausgeführt. Der Füllschlauch 20 hat einen deutlich größeren Innendurchmesser als der Füllschlauch 19 der Ausführung nach Fig. 9. Dieser Innendurchmesser des Füllschlauchs 20 ist so bemessen, dass der Füllschlauch 20 dicht auf die Außenwandung der Abfüllnadel 16 aufgeschoben werden kann. In diesem Fall kann ein Überstand der inneren Silikon-Schlauchschicht 2 der Abfüllnadel 16 über die Versteifungs-Schlauchschicht 3 am Konnektorende 18 entfallen, wie in der Fig. 10 dargestellt.

Bei der Ausführung nach Fig. 11 kommt ein Silikonkonnektor 21 zum Einsatz, wie dieser grundsätzlich aus der DE 10 2011 076 938 A1 bekannt ist.

Alternativ zur Ausführung nach Fig. 10 kann auch die am Konnektorende 18 in einem entsprechend großen Überstandsbereich d freigelegte innere Silikon-Schlauchschicht 2 in einen entsprechend dem Durchmesser angepassten Füllschlauch nach Art des Füllschlauchs 20 eingeschoben werden, was in der Zeichnung nicht dargestellt ist. Ein Innendurchmesser dieser Variante des Füllschlauchs 20 entspricht dann einem Außendurchmesser der inneren Silikon-Schlauchschicht 2.

Die innere Silikon-Schlauchschicht 2 kann bei der Anordnung nach Fig. 11 dichtend mit einem inneren Anschlagselement 22 des Konnektors 21 abschließen, so dass eine fluiddichte Verbindung der inneren Silikon-Schlauchschicht 2 hin zum Konnektor 21 gegeben ist.

Durch entsprechende Ausgestaltungen der Konnektierung, die vorstehend im Zusammenhang mit den Fig. 9 bis 11 beschrieben wurden, kann erreicht werden, dass sämtliche Fluidkanaloberflächen der Schlauchschicht vom Abfüllbehälter bis zum Nadelende 17 aus Silikon bzw. Silikonkautschuk bestehen.

Bei einer nicht dargestellten Ausführung der Abfüllnadel 16 kommt ein chemischer Haftvermittler zum Einsatz. In diesem Fall kann auf Haftvermittlungskörper nach Art der Haftvermittlungskörper 5 auch verzichtet werden. Alternativ ist es möglich, eine Haftvermittlung zwischen den Schlauchschichten 2 und 3 über eine Kombination von Haftvermittlungskörpern nach Art der Haftvermittlungskörper 5 und einer chemischen Haftvermittlungskomponente zu schaffen.

## Patentansprüche

1. Abfüllnadel (16) zur Verwendung in einem Schlauchset zum Abfüllen eines fließfähigen Mediums, insbesondere eines Pharmazeutikums,
- mit einem Nadelende (17) zum zeitweiligen Einführen in einen Abfüllbehälter,
- mit einem Konnektorende (18) zum Konnektieren der Abfüllnadel (16) mit einer Fülleinrichtung (19a), über die das Medium der Abfüllnadel (16) zuführbar ist,
- mit einer ein inneres Schlauchlumen (8) vorgebenden inneren Schlauchschicht (2) aus Silikon, und
- eine das innere Schlauchlumen (8) umgebende äußere Versteifungs-Schlauchschicht (3) aus einem Kunststoff-Versteifungsmaterial,
- wobei die Versteifungs-Schlauchschicht (3) aus Polypropylen, Polyethylen, Polycarbonat oder Polysulfon gefertigt ist und
- wobei die Abfüllnadel (16) insgesamt ausschließlich aus Kunststoffmaterial besteht.

2. Abfüllnadel nach Anspruch 1, **gekennzeichnet durch** einen Haftvermittler (4) zwischen der inneren Schlauchschicht (2) und der Versteifungs-Schlauchschicht (3).

3. Abfüllnadel nach Anspruch 2, **dadurch gekennzeichnet, dass** der Haftvermittler (4) Haftvermittlungskörper (5) aufweist, die die beiden Schlauchschichten (2, 3) mechanisch miteinander verbinden.

4. Schlauchset zum Abfüllen eines fließfähigen Mediums
- mit einer Abfüllnadel (16) nach einem der Ansprüche 1 bis 3, und
- mit einer Fülleinrichtung (19a) zum Zuführen des Mediums zur Abfüllnadel (16).

5. Schlauchset nach Anspruch 4, **dadurch gekennzeichnet, dass** die innere Schlauchschicht (2) der Abfüllnadel (16) auf eine KonnektorKomponente (19) zum Verbinden der Abfüllnadel (16) mit der Fülleinrichtung (19a) aufgestülpt ist.

6. Schlauchset nach Anspruch 4, **dadurch gekennzeichnet, dass** die innere Schlauchschicht (2) der Abfüllnadel (16) in eine Konnektor-Komponente (20; 21) zum Verbinden der Abfüllnadel (16) mit der Fülleinrichtung (19a) eingeschoben ist.

## Claims

1. Filling needle (16) for use in a tube set for transferring a flowable medium, in particular a pharmaceutical product,
- having a needle end (17) for temporary insertion into a filling vessel,
- having a connector end (18) for connecting the filling needle (16) to a filling device (19a) via which the medium can be supplied to the filling needle (16),
- having an inner tube layer (2) made of silicone, defining an inner tube lumen (8), and
- an outer reinforcing tube layer (3) made of a plastic reinforcing material, encasing the inner tube lumen (8),
- wherein the reinforcing tube layer (3) is made of polypropylene, polyethylene, polycarbonate or polysulfone and
- wherein the filling needle (16) on the whole consists of plastic, exclusively.

2. Filling needle according to Claim 1, **characterized by** a bonding agent (4) between the inner tube layer (2) and the reinforcing tube layer (3).

3. Filling needle according to Claim 2, **characterized in that** the bonding agent (4) has bonding bodies (5), which mechanically bond the two tube layers (2, 3).

4. Tube set for transferring a flowable medium
- having a filling needle (16) according to one of the Claims 1 to 3, and
- having a filling device (19a) for supplying the medium to the filling needle (16).

5. Tube set according to Claim 4, **characterized in that** the inner tube layer (2) of the filling needle (16) is slid over a connector component (19) to connect the filling needle (16) to the filling device (19a).

6. Tube set according to Claim 4, **characterized in that** the inner tube layer (2) of the filling needle (16) is slid into a connector component (20; 21) to connect the filling needle (16) to the filling device (19a).

## Revendications

1. Aiguille de remplissage (16) destinée à être utilisée dans un ensemble de tuyaux pour le remplissage d'un milieu fluide, en particulier d'un produit pharmaceutique
- ayant une extrémité d'aiguille (17) pour l'insertion temporaire dans un récipient de remplissage,
- comportant une extrémité de connecteur (18) pour relier l'aiguille de remplissage (16) à un dispositif de remplissage (19a) par lequel le fluide peut être amené à l'aiguille de remplissage (16),
- une couche de tuyau (2) en silicone définissant une lumière de tuyau (8) intérieure, et
- une couche externe de tuyau raidisseur (3) d'un matériau plastique raidisseur entourant la lumière de tuyau (8) intérieure,
- dans laquelle la couche de tuyau raidisseur (3) est fabriquée en polypropylène, polyéthylène, polycarbonate ou polysulfone, et
- dans laquelle l'aiguille de remplissage (16) dans son ensemble est constituée exclusivement de matière plastique.

2. Aiguille de remplissage selon la revendication 1, **caractérisée par** un agent adhésif (4) entre la couche de tuyau intérieure (2) et la couche de tuyau raidisseur (3).

3. Aiguille de remplissage selon la revendication 2, **caractérisée en ce que** l'agent adhésif (4) comprend des corps d'agent adhésif (5) qui relient mécaniquement les deux couches de tuyaux (2, 3) l'une à l'autre.

4. Ensemble de tuyaux pour le remplissage d'un milieu fluide
- avec une aiguille de remplissage (16) selon l'une quelconque des revendications 1 à 3, et
- avec un dispositif de remplissage (19a) pour amener le milieu à l'aiguille de remplissage (16).

5. Ensemble de tuyaux selon la revendication 4, **caractérisé en ce que** la couche de tuyau intérieure (2) de l'aiguille de remplissage (16) est montée sur un élément connecteur (19) pour relier l'aiguille de remplissage (16) au dispositif de remplissage (19a).

6. Ensemble de tuyaux selon la revendication 4, **caractérisé en ce que** la couche de tuyau intérieure (2) de l'aiguille de remplissage (16) est insérée dans un élément connecteur (20 ; 21) pour relier l'aiguille de remplissage (16) au dispositif de remplissage (19a).
